# FASCICULE DE BREVET EUROPEEN

(11) **EP 4 072 273 B1**
(45) Date de publication et mention de la délivrance du brevet: **27.08.2025**
(21) Numéro de dépôt: 20845178.1
(22) Date de dépôt: 11.12.2020
(51) Int. Cl.: A01H 1/08, A01H 6/14

(54) **PROCEDE D'HAPLODIPLOIDISATION DE PLANTES DE TOURNESOL**
DOPPELHAPLOIDVERFAHREN FÜR SONNENBLUMENPFLANZEN
DOUBLE-HAPLOID METHOD FOR SUNFLOWER PLANTS

(30) Priorité: 13.12.2019 FR 1914390
(43) Date de publication de la demande: 19.10.2022
(73) Titulaire: RAGT 2N, 12000 Rodez (FR); Mas Seeds, 40280 Haut Mauco (FR)
(72) Inventeur: BODIN, Manuelle, 29600 MORLAIX (FR); FLORIN, Christelle, 40500 SAINT SEVER (FR); GAILLARD, Antoine, 40280 SAINT PIERRE DU MONT (FR); LUCAS, Olivier, 12000 RODEZ (FR); MADRE, Cécile, 29250 SAINT POL DE LEON (FR); PHILIPPOT, Murielle, 29250 SAINT POL DE LEON (FR); ROMESTANT, Michel, 12850 SAINTE RADEGONDE (FR); TARDIN, Marie-Claire, 12510 OLEMPS (FR)
(74) Mandataire: Regimbeau
(86) Numéro de dépôt international: PCT/FR2020/052389
(87) Numéro de publication internationale: WO 2021/116619

(56) Documents cités:
- M TODOROVA ET AL: "Doubled haploid production of sunflower (Helianthus annuus L.) through irradiated pollen-induced parthenogenesis", EUPHYTICA, vol. ,97, no. 249, 1 January 1997 (1997-01-01), pages 249 - 254, XP055737707
- PASCALE GELEBART ET AL: "Obtention de plantes haploïdes par culture in vitro d'ovaires et d'ovules non fécondés de tournesol (Helianthus annuus L.)", AGRONOMIE, vol. 7, no. 2, 1 January 1987 (1987-01-01), pages 81 - 86, XP055737608, DOI: https://doi.org/10.1051/agro:19870202

## Description

La présente invention concerne une méthode d'haplodiploïdisation de plantes de tournesol.

L'haplodiploïdisation, ou technique de production des haploïdes doublés, est une méthode utilisée pour fixer génétiquement, c'est-à-dire rendre homozygote, du matériel végétal aux fins de création variétale, et ce, plus rapidement que par autofécondations successives.

Cette technique repose sur l'obtention de plantes haploïdes (n) à partir des organes mâles (pollen) ou femelles (ovules) de la plante, puis sur le doublement du stock chromosomique.

L'haploïdie (n) est une situation génétique naturelle et fugace puisque seuls les gamètes présentent cet état génétique et que leur durée de vie est courte. En doublant le nombre de chromosomes dans ces cellules, on restaure l'état diploïde (2n) normal. Les deux copies de chaque gène étant identiques, on obtient des plantes diploïdes homozygotes pour chaque locus.

L'état haploïde étant instable, l'individu régénéré est parfois diploïde, et ce via doublement spontané du stock chromosomique. Pour le blé, on peut compter 20 à 25 % d'haploïdes doublés spontanément, 60 à 65 % chez l'orge, par exemple.

En l'absence de doublement spontané ou pour assurer un % plus élevé, cette étape de doublement du stock chromosomique permettant de passer à l'état diploïde se fait traditionnellement par un traitement chimique, fréquemment la colchicine. Ce traitement à la colchicine a ainsi pour but de doubler le stock chromosomique. La colchicine bloque les divisions cellulaires, c'est à dire qu'elle stoppe la mitose à la phase métaphase en bloquant le fuseau de microtubules. Son intérêt est de passer de n à 2n pour obtenir une plante fertile et viable.

Cela mène à l'obtention de plantes diploïdes (2 n), homozygotes pour l'ensemble des locus du génome ; le matériel végétal est ainsi « fixé », appelé lignée, et utilisable, par exemple, en croisements afin de produire des hybrides. Une lignée fixée, ou lignée pure, est totalement homozygote. Elle transmet donc à 100% de sa descendance exactement le même patrimoine génétique.

L'haplodiploïdisation est une technique rapide pour obtenir des lignées pures en une seule étape au lieu de 7 à 8 générations dans le cas d'autofécondations. Avec cette méthode, la durée globale d'un cycle de sélection variétale est ainsi raccourcie de 3 à 5 ans.

Comme indiqué précédemment, l'obtention des plantes haploïdes peut se faire par culture in vitro des cellules destinées à fournir les cellules reproductrices ou gamètes. S'il s'agit de gamètes mâles, on parle d'androgenèse. S'il s'agit de gamètes femelles, c'est la gynogenèse.

L'androgenèse a été, historiquement, la première voie d'obtention d'haploïdes in vitro. Les plantes haploïdes ont été obtenues par culture d'anthères. Plus récemment, la culture de microspores a été mise au point et tend à se développer. La voie androgénique a été appliquée avec succès sur le colza, l'orge, le blé, l'asperge, le piment et l'aubergine, entre autres. Pour le cas du tournesol, cela a aussi été décrit, mais donne des résultats insatisfaisants (Cf Todorova et al, Biotechnol. & Biotechnol. Eq. 11 (4) : 27-30,1997*).*

L'haploïdie induite par gynogenèse consiste à mettre en culture in vitro des ovules ou des ovaires prélevés sur la plante avant fécondation. Des plantes haploïdes ont pu être obtenues chez l'orge, le tabac, le blé, le riz, le maïs et la betterave. Alternativement, la gynogénèse peut aussi être induite par pollinisation avec du pollen non fonctionnel (dénaturé et/ou interspécifique) ou fonctionnel (cas particulier du maïs) qui agit comme agent inducteur du développement haploïde dans la graine. Mis à part dans le cas particulier du maïs, on obtient alors un embryon immature, dépourvu des tissus nourriciers de la graine.

Le tournesol, (*Helianthus annuus,L.*), appartient à la famille des Astéracées. C'est une plante annuelle. L'inflorescence caractéristique des Astéracées (composées) est un capitule qui comprend, insérées sur son réceptacle charnu, un grand nombre de petites fleurs (fleurons) tubulées hermaphrodites disposées en spirale et un rang périphérique de fleurs stériles ligulées, avec une languette très colorée.

Chaque fleuron tubulé, fertile présente un calice modifié (écailles), 5 pétales soudés (tube), un androcée synanthéré avec 5 anthères introrses soudées formant un étui, les filets étant séparés et insérés à la base de la corolle. L'ovaire infère comprend 2 carpelles mais un seul ovule, il est surmonté par le style qui traverse l'étui formé par les anthères, et qui se termine par 2 stigmates.

La floraison est centripète, les fleurons de 3 ou 4 rangs successifs s'ouvrent en même temps, les cercles périphériques fleurissant d'abord. Ce sont ainsi les fleurons sur les bords extérieurs qui fleurissent en premier. L'ensemble de la floraison de toutes les fleurs du capitule peut s'étaler sur une quinzaine de jours.

L'ouverture d'une fleur commence le matin, les filets des étamines (organes mâles) s'allongent rapidement et la colonne formée par les anthères dépasse de la corolle puis leur déhiscence se produit, le pollen (gamètes mâles) se retrouvant alors à l'intérieur de la colonne.

L'élongation du style (organe femelle), dans la colonne pousse un peu de pollen à l'extrémité du tube formé par les anthères, il peut alors être récupéré par des butineurs. En fin d'après-midi, les 2 stigmates, encore réunis, commencent à sortir du tube constitué par les anthères et le matin suivant, ils se séparent et s'enroulent. La face interne réceptrice de chaque stigmate, alors à maturité, est bien exposée pour retenir du pollen et permettre éventuellement sa germination. La face réceptrice des stigmates possède des papilles gluantes courtes et denses.

La protandrie de la fleur (maturité des pièces fertiles mâles qui précède la maturité des pièces fertiles femelles) est dépendante de la différence de vitesse de croissance (contrôlée par l'intervention d'hormones végétales) des filets et du style.

Comme évoqué précédemment en ce qui concerne l'haploïdisation, l'induction d'haploïdes à partir d'ovules est également possible par pollinisation avec du pollen dénaturé par irradiation.

Aussi, dans le cas du tournesol, la gynogénèse peut être obtenue par une induction via une pollinisation avec du pollen inactivé, par exemple irradié, car la simple mise en culture, sans inducteur, des ovules ou ovaires non fécondés ne permet pas d'obtenir d'embryons haploïdes (cf Todorova et al, HELIA, 22, Nr 31, 66 49-56, 1999 *;* Todorova et al, Euphytica, 97: 249-254, 1997).

Irradier du pollen le rend inactif mais néanmoins capable d'initier des divisions cellulaires de l'ovule. Ceci permet l'obtention d'une plantule sans fécondation donc également avec le seul lot de chromosomes maternels. On parle de parthénogénèse induite.

Ces techniques sont cependant très lourdes et complexes car elles impliquent une collecte du pollen, son traitement par irradiation aux rayons gamma dans des installations dédiées à cet effet ; puis une étape de pollinisation avec ledit pollen ainsi traité.

Alternativement, la gynogénèse chez le tournesol existe aussi par culture d'ovaires prélevés avant floraison. L'induction de l'embryon haploïde est réalisée par culture d'ovaires ou d'ovules *in vitro* à l'aide d'un milieu de culture inducteur après extraction de ceux-ci(Gelebart et al, Agronomie, EDP Sciences, 7 (2), 81-86, 1987). Dans ce cas, l'embryon est formé hors de la plante.

Cette technique est très fastidieuse, lourde et très peu d'équipes ont réussi à la reproduire de manière fiable et répétée.

En ce qui concerne la production d'haploïdes de tournesol par androgénèse, les rendements obtenus et les défauts induits font que cette technique n'est pas, ou que très peu, utilisée.

Ainsi, pour le cas du tournesol, il ressort donc qu'il existe un besoin en une technique d'haploïdisation, y compris d'haplodiploïdisation, qui d'une part donne de bons résultats en termes de rendements et des plantes exemptes de défauts, et d'autre part soit de mise en œuvre facile en évitant des manipulations complexes.

Les inventeurs de la présente invention ont ainsi découvert, de manière surprenante, que dans le cas de la plante de tournesol, la castration des fleurs par suppression des organes mâles permet l'obtention, in planta, d'embryons haploïdes immatures, et ce indépendamment de toute pollinisation, que ce soit avec du pollen fonctionnel ou non fonctionnel.

### RESUME DE L'INVENTION

Ainsi la présente invention concerne une méthode de production de plante ou plantule haploïde ou haploïde doublée de tournesol (*Helianthus annuus*) comprenant les étapes suivantes :
- a) Fourniture d'une plante de tournesol diploïde, au stade de floraison du capitule,
- b) Castration avant émission du pollen, en particulier par ablation des anthères des fleurs tubulées,
- c) prélèvement d'un embryon haploïde immature formé dans l'ovaire non fécondé des fleurs tubulées, sur la plante,
- d) Sauvetage de l'embryon haploïde immature in vitro,
- e) Obtention de plante ou plantule de tournesol haploïde ou haploïde doublée,
la méthode étant caractérisée par l'absence de tout contact des fleurs tubulées avec du pollen de tournesol (*Heliantus annuus*)*.*

De manière optionnelle, la méthode selon l'invention peut comprendre une étape de doublement chromosomique, en particulier par la mise en œuvre d'un agent de doublement chromosomique, si nécessaire.

Une telle étape de doublement chromosomique est en effet optionnelle car un doublement chromosomique spontané est possible et dans un tel cas de figure, une telle étape de doublement chromosomique n'est pas nécessaire.

S'il n'y a pas de doublement chromosomique spontané chez l'embryon obtenu et que celui-ci est voulu et souhaité, alors l'étape en question devient nécessaire.

Alternativement, cette étape est aussi optionnelle, et donc non réalisée, dans le cas ou même en cas de non doublement spontané, celui-ci n'est pas voulu car on cherchera à obtenir une plante haploïde.

Lorsque le doublement chromosomique est nécessaire et voulu et qu'il ne se produit pas spontanément, on va donc prévoir une étape de doublement chromosomique, en particulier par la mise en œuvre d'un agent de doublement chromosomique.

Une telle étape de doublement chromosomique peut être réalisée sur l'embryon, donc après l'étape d) et avant l'étape e).

Une telle étape de doublement chromosomique peut aussi être réalisée sur la plantule, donc après l'étape f).

Lorsque réalisée sur la plantule, l'étape de doublement chromosomique est réalisée sur les racines ou sur le méristème apical.

De manière avantageuse, la plantule de tournesol haploïde doublée est homozygote.

La méthode ici décrite peut comprendre une étape supplémentaire d'autofécondation des plantes haploïdes doublées obtenues afin de produire des graines homozygotes de tournesol. Les graines ainsi produites par autofécondation permettent aussi de disposer d'une descendance des plantes obtenues par la méthode de l'invention. Ces graines permettent de propager la plante de tournesol par simple semis et croissance et de l'utiliser quand nécessaire pour des programmes de croisement et de sélection.

Alternativement, la méthode ici décrite pourra aussi comprendre une étape supplémentaire de fécondation contrôlée et dirigée des plantes haploïdes doublées obtenues, et ce par du pollen d'une autre plante de tournesol pour créer une descendance directement utilisable dans un programme de sélection variétale.

### DEFINITIONS

Dans le cadre de la méthode selon l'invention, le terme tournesol fait référence à la plante *Hélianthus annuus* L.

Une plante ou plantule haploïde possède un seul jeu de chromosomes et le nombre réduit de chromosomes (n) dans la plante haploïde est égal à celui du gamète.

Une plante diploïde possède deux jeux de chromosomes et le nombre de chromosomes (2n) est égal à celui du zygote.

Une plante haploïde doublée, plantule haploïde doublée, ou une cellule haploïde doublée est celle qui est développée par le doublement d'un ensemble haploïde de chromosomes.

Une plante ou une graine obtenue à partir d'une plante haploïde doublée et qui est autonome, peu importe le nombre de générations, peut encore être identifiée comme une plante haploïde doublée.

Une plante haploïde doublée est considérée comme une plante homozygote. Une plante est considérée haploïde doublée si elle est fertile, même si toute la partie végétative de la plante ne comprend pas les cellules avec le jeu de chromosomes doublé. Par exemple, une plante sera considérée comme une plante haploïde doublée si elle contient des gamètes viables, même si elle est chimérique.

Un "embryon haploïde doublé" est un embryon comportant une ou plusieurs cellules contenant 2 ensembles de chromosomes homozygotes.

Les expressions "contact", "entre en contact avec" ou "mis en contact avec" peuvent signifier "contact direct" ou "contact indirect".

Par exemple, le milieu comprenant un agent de doublement chromosomique peut être en contact direct avec la cellule haploïde ou le milieu contenant l'agent de doublement peut être séparé de la cellule haploïde par du papier filtre, des tissus végétaux ou d'autres cellules, de sorte que l'agent de doublement est transféré à travers le filtre papier ou des cellules à la cellule haploïde.

Le terme "milieu" comprend les composés à l'état liquide, gazeux ou solide.

Tel qu'utilisé ici, le terme "plante" inclut les plantes et les plantules ainsi que leur descendance. Le terme peut être utilisé au singulier ou au pluriel indifféremment.

Une plantule ou jeune pousse est une jeune plante ne comportant que quelques feuilles. Issue de l'embryon d'une graine, son développement commence avec la germination de la graine.

"Cellule végétale", telle qu'utilisée ici, comprend, sans limitation, les graines, les cultures en suspension, les embryons, les régions méristématiques, le tissu calleux, les feuilles, les racines, les pousses, les gamétophytes, les sporophytes, le pollen et les microspores.

L'expression « stade de floraison du capitule » au sens de la présente invention signifie qu'une partie au moins des fleurons (c'est-à-dire au moins un fleuron) que contient le capitule sont ouverts, c'est-à-dire sont au stade de floraison et présentent des pièces fertiles
L'expression « embryon immature » au sens de la présente invention signifie qu'une structure embryonnaire, haploïde ou diploïde, s'est développée dans l'ovaire du fleuron et qu'il présente un niveau de développement plus précoce que l'embryon mature de la graine, nécessitant son extraction pour culture sur milieu nutritif in vitro.

### DESCRIPTION DETAILLEE

L'étape a) de la méthode selon l'invention comprend donc la fourniture d'une plante de tournesol diploïde au stade de floraison du capitule. Une telle plante peut s'obtenir par le semis de graines de tournesol, soit de graines homozygotes, i.e. lignées, soit de graines hétérozygotes, ie hybrides, par exemple hybride F1.

Selon un mode de réalisation de l'invention, la plantule de l'étape a) est homozygote ou hétérozygote.

Le semis est réalisé selon les techniques classiques et connues d'un homme du métier, semis en godets puis rempotage en pots contenant un substrat adapté, tel qu'un terreau commercial de semis pour les godets puis terreau commercial de culture pour les pots, par exemple.

De manière avantageuse le semis et la croissance des plantules est réalisé sous serre et en particulier à une température de l'ordre de 18 à 20°C et avec une photopériode de l'ordre de 12 à 20 heures, en particulier 16 heures environ et ce afin que les capitules soient bien développés.

Typiquement, on prévoira un délai de quelques semaines, par exemple 8 à 10 semaines entre le semis et la floraison des plants, c'est-à-dire l'obtention de plantes au stade de floraison du capitule.

Cette étape permet d'obtenir des plants de tournesol en début de floraison du capitule, c'est-à-dire avec les premières fleurs tubulées qui s'ouvrent.

C'est l'étape b) de la méthode selon l'invention qui prend son importance car comme indiqué précédemment, les inventeurs ont pu remarquer que la castration des organes mâles des fleurs permet d'induire le développement d'embryons haploïdes dans les ovules non fécondés de certaines de ces fleurs.

Ainsi cette étape b) de castration comprend les sous-étapes suivantes et se déroule de la manière suivante.

La castration se déroule sur plusieurs jours et peut débuter, le premier jour, lorsque les capitules sont bien ouverts, en l'ablation des fleurs ligulées situées à la périphérie du capitule. Cette étape est optionnelle.

La castration peut comprendre ainsi une première étape de retrait des fleurs tubulées déjà ouvertes situées sur le contour du capitule.

Ce retrait se réalise manuellement, par exemple à l'aide d'une pince à épiler ou d'un outil similaire.

Il convient de prendre garde à bien ôter l'ovaire en partie basse du fleuron.

Par la suite, chaque jour, est procédé à l'ablation des anthères des fleurs tubulées sorties le matin même.

Il convient de prendre garde à n'ôter que les anthères et ne pas endommager ni enlever le pistil.

Cette étape d'ablation des anthères peut être réalisée à l'aide d'une pince à épiler par exemple ou à l'aide de tout autre outil adéquat bien connu de l'homme du métier.

Avant l'ablation des anthères, on pourra procéder à un lavage des fleurons afin d'éliminer toute trace de pollen qui pourrait être tombé des anthères proximales.

De manière avantageuse, cette étape de castration par ablation des anthères peut être répétée quotidiennement sur plusieurs jours au fur et à mesure du développement des fleurs tubulées et de l'ouverture de ces fleurs au stade mâle.

Typiquement, ces étapes de castration sont répétées quotidiennement pendant 2 à 7 jours, particulièrement 3 à 4 jours.

Les fleurs tubulées non ouvertes après la fin de la castration du capitule, par exemple après 10 jours, après 9 jours, après 8 jours, après 7 jours, ou après 6 jours, ou après 5 jours, ou après 4 jours, ou après 3 jours voire encore après 2 jours, à compter du premier jour de castration par ablation des anthères, sont éliminées. Cela est aussi réalisé à l'aide d'une pince à épiler ou d'un outil comparable.

Durant ces étapes de castration, les capitules peuvent être recouverts d'un sachet maintenu par un lien pour éviter tout risque de pollinisation parasite.

De manière avantageuse, l'étape, ou les étapes, de castration est ou sont réalisées sous serre.

De manière générale, toutes les précautions pourront être prises pour éviter les pollinisations et/ou contaminations polliniques. En particulier les plantes en fleurs seront éloignées des plantes en cours de castration.

Les outils utilisés pour la castration, tels que pince à épiler utilisée pour l'ablation des anthères, seront régulièrement rincés à l'eau en cours de castration et lavés à l'eau savonneuse après les étapes de castration quotidiennes et avant la reprise des étapes de castration du lendemain.

Après les étapes de castration et d'élimination des fleurs tubulées non ouvertes, le capitule de tournesol ne contient plus que des fleurs ne présentant plus d'organe mâle ni de pollen.

Les plants portant de tels capitules sont ainsi laissés en pots, sous serre, en conditions de culture classiques de température et de photopériode, comme indiqué précédemment.

Après 3 à 25 jours, voire 7 à 20 jours, en particulier entre 10 et 18 jours, plus particulièrement entre 11 et 15 jours de culture de ces plants, les ovaires sont récoltés.

L'étape c) de prélèvement de l'embryon immature contenu et formé dans la graine immature, *in planta,* peut être réalisée sous la loupe binoculaire.

Il est important de noter que dans la méthode selon la présente invention, l'embryon haploïde immature est formé dans l'ovaire non fécondé, sur la plante, c'est-à-dire *in planta.*

En effet selon d'autres techniques, l'ovaire ou l'ovule est prélevé et mis en culture dans un milieu stimulant et inducteur et l'embryon se forme in vitro. C'est ce qui est décrit dans *Gelebart* et al., or ce n'est pas le cas dans la présente invention puisque l'embryon est formé dans l'ovaire non fécondé des fleurs tubulées, et ce sur la plante même.

Cette étape de prélèvement se réalise ainsi selon des techniques connues de l'homme du métier.

En l'espèce, les ovaires des capitules sont prélevés avant d'être disposés dans un récipient individuel de type flacon d'Erlenmeyer ou Becher par exemple.

Les ovaires sont généralement lavés et désinfectés à l'aide de solutions adéquates du type hypochlorite de calcium dilué, par exemple.

Les ovaires désinfectés peuvent être ensuite rincés abondamment à l'eau distillée stérile.

L'embryon immature haploïde peut être extrait de l'ovaire par incision en conditions stériles, et placé sur du milieu de culture in vitro.

Les inventeurs ont ainsi constaté, de manière surprenante, lors des étapes exploratoires d'induction de gynogénèse classique par pollen irradié, que les graines des capitules de tournesol castrés pouvaient contenir des embryons haploïdes immatures - mais viables - et ce malgré une absence de pollinisation ou d'induction par pollen irradié. La méthode selon l'invention permet ainsi d'éviter l'opération fastidieuse de collecte de pollen, et de traitement de ce pollen.

La méthode selon l'invention est ainsi caractérisée par l'absence de contact des fleurs tubulées avec du pollen de tournesol (*Helianthus annuus*); et que ce soit du pollen fonctionnel ou non fonctionnel, par exemple irradié, de tournesol (*Helianthus annuus*).

Les inventeurs ont ainsi constaté dans des expériences comparatives mettant en œuvre une étape de mise en contact des fleurons castrés avec du pollen fonctionnel ou non fonctionnel, en particulier du pollen irradié, que le taux d'obtention d'embryons ne changeait pas de manière significative et qu'un tel contact, présenté dans l'art antérieur comme une condition indispensable d'induction et d'obtention de l'embryon, n'était en fait pas nécessaire.

Les embryons immatures haploïdes, ainsi extraits des graines de fleurs tubulées castrées, font l'objet d'une étape de sauvetage d'embryons. Cette étape est en effet nécessaire car du fait de l'absence de fécondation, il n'y a pas de développement des tissus nourriciers de la graine et donc pas survie hors in vitro.

Par exemple, ils peuvent être disposés sur, ou mis en contact avec, un milieu de culture solide pour réaliser l'étape d) de sauvetage d'embryon.

La méthode selon l'invention prévoit dans l'étape d) un sauvetage d'embryon immature par l'utilisation d'une technique connue de sauvetage d'embryons. Le sauvetage d'embryon est réalisé en mettant, par exemple, en contact un embryon avec un milieu contenant des nutriments. Les phytohormones peuvent être incluses ou non dans le support de sauvetage d'embryon.

Typiquement, les embryons immatures sont placés dans un milieu adéquat pour sauvetage d'embryon. Ces techniques sont connues de l'homme du métier. On peut citer par exemple un milieu de Murashige et de Skoog (MS, 1962) avec 2% de saccharose et 0.8% d'agar au pH 5.6-5.7 (cf Jambhulkar, S. J., 1995. Rapid cycling through immature embryo culture in sunflower (Helianthus annuus L). Helia, 18(22): 45-50.).

Les boîtes de Pétri peuvent être scellées, par exemple avec du Parafilm^{®}, pour prévenir la perte d'humidité et placées à l'obscurité dans un premier temps pendant environ 5 à 10 jours, en particulier environ 7 jours, puis à la lumière alternante jour/nuit (alternance 16h/8h jour/nuit par exemple) aux environs de 25°C jusqu'à la production de plantules.

Dans les 1 à 2 semaines de culture sur le milieu adéquat de sauvetage d'embryons, les embryons en développement donnent lieu à des plantules pourvues de racines (environ 2-6 cm et ramification) et d'une tige (environ 3-5 cm) avec quelques feuilles (environ 1 à 5 cm).

Après cette étape de sauvetage d'embryons, une analyse de la ploïdie permet de vérifier si les cellules des plantes sont haploïdes ou diploïdes.

Les cellules haploïdes (d'embryons, de graines, de plantes, etc.) peuvent être identifiées par plusieurs méthodes, telles que le comptage chromosomique, la mesure de la longueur des cellules de garde (sur feuilles des plantes) ou l'utilisation d'un cytomètre de flux.

Une étape de marquage moléculaire peut être réalisée sur les embryons ou plantules haploïdes doublées afin de vérifier que la diploïdie est bien due à un doublement chromosomique, et donc qu'il s'agit d'une plantule ou embryon homozygote, et ne correspond pas à un échappement, c'est-à-dire due à une autofécondation menant à un individu hétérozygote.

Selon un mode de réalisation de l'invention on obtient une plante (ou plantule selon la taille) haploïde doublée (haplodiploïde) en mettant en contact un embryon haploïde avec un agent de doublement et on obtient un embryon, ou une plante haploïde doublée.

En effet, il peut y avoir un doublement spontané des chromosomes et on peut ainsi obtenir des plantes, embryons ou plantules, dont les cellules sont diploïdes sans avoir besoin de procéder à un traitement chimique de doublement chromosomique.

Ainsi les cellules haploïdes, les embryons haploïdes, les graines haploïdes, les plantules haploïdes ou les plantes haploïdes peuvent être traités, si nécessaire au cas où le doublement chromosomique n'est pas spontané, avec un agent de doublement chromosomique. Les plantes homozygotes peuvent ainsi être régénérées à partir de cellules haploïdes en mettant en contact les cellules haploïdes, telles que les cellules d'embryons haploïdes, ou les embryons entiers haploïdes, ou les plantes entières haploïdes, avec des agents de doublement des chromosomes.

Ainsi la méthode selon l'invention est caractérisée en ce qu'elle comprend une étape de traitement de doublement chromosomique.

Selon un mode de réalisation, la méthode est ainsi caractérisée en ce que le traitement de doublement chromosomique comprend l'application d'un agent de doublement chromosomique au niveau de l'embryon obtenu à l'étape d).

Selon un mode de réalisation, la méthode est ainsi caractérisée en ce le traitement de doublement chromosomique comprend l'application d'un agent de doublement chromosomique au niveau du méristème apical de la plantule obtenue à l'étape e).

Selon un autre mode de réalisation, la méthode est ainsi caractérisée en ce le traitement de doublement chromosomique comprend l'application d'un agent de doublement chromosomique au niveau des racines de la plantule obtenue à l'étape e).

La présente description divulgue aussi une plante ou plantule de tournesol haploïde doublée obtenue par une méthode selon l'invention comprenant une étape de traitement de doublement chromosomique.

Des méthodes typiques impliquent la mise en contact des cellules avec des agents doublant tels que par exemple : la colchicine, des agents anti-microtubules ou des herbicides anti-microtubules, le pronamide, l'oxyde nitreux ou tout autre inhibiteur mitotique par exemple afin de créer des cellules haploïdes doublées homozygotes.

La quantité de colchicine utilisée dans le milieu est généralement de 0,01% à 0,2% ou environ 0,05%. D'autres agents peuvent être utilisés avec les inhibiteurs mitotiques pour améliorer l'efficacité du doublement chromosomique.

L'agent de doublement peut entrer en contact avec l'embryon ou la plante à différents moments. Si l'embryon est isolé, l'agent de doublement peut entrer en contact immédiatement après l'isolement et avant la germination. Si l'embryon est contenu dans la graine, il peut entrer en contact avec l'agent de doublement à tout moment. S'il s'agit d'intervenir sur la plante, le contact est réalisé sur tout ou partie de la plantule haploïde.

La durée de contact entre l'agent de doublement chromosomique peut varier. Le contact peut durer moins de 24 heures, par exemple 4 à 12 heures, à environ une semaine. La durée de contact est généralement d'environ 24 heures à 2 jours.

Les cellules haploïdes peuvent entrer en contact avec l'agent de doublement au stade de l'embryon, au stade de la graine mature, au stade de semis, ou préférentiellement au stade de la plantule.

Dans ce dernier cas, le traitement peut viser les racines ou le méristème apical.

Dans le cas de traitement des racines de plantule, on pourra prévoir un traitement durant 1 à 10 heures, en particulier 2 à 5 heures, avec une solution de colchicine à 200 à 300 mg/L, particulièrement 300 à 500 mg/L.

Dans le cas de traitement du méristème apical, on pourra prévoir un traitement durant 3 à 8 heures, en particulier environ 5 heures et avec une solution de colchicine à 1000 à 2000 mg/L, en particulier 1500 mg/L. Typiquement, le traitement pourra consister en l'application d'une goutte d'agent de doublement chromosomique, en particulier la colchicine, au niveau du méristème apical.

Après le doublement des chromosomes, l'embryon ou la plante haploïde doublé contiendra 2 copies de chromosomes d'origine maternelle. L'efficacité du processus d'obtention de plantes haploïdes doublées à partir d'embryons haploïdes peut être supérieure à 10%, 20%, 30%, 50%, 60%, 70%, 80% ou 90%.

Des méthodes de doublement de chromosomes sont décrites dans la littérature, par exemple par Kasha (2005) Haploids in crop improvement II. Biotechnologie in Agriculture and forestry, vol. 56. Chap 1.7 pp 123-152.

Les plantes de tournesol haploïdes ou haploïdes doublées ainsi obtenues pourront être soumises à une étape supplémentaire d'acclimatation.

Cette étape permet à la plantule *in vitro* de s'installer progressivement dans un nouvel environnement de culture, différent de la culture *in vitro* par le climat, la température, l'humidité et les ressources.

En effet il s'agit pour le jeune plant de passer d'un milieu de culture solide in vitro, à un milieu de culture de type terreau et terre dans une serre et ainsi au plus près des conditions réelles de culture pour un tournesol.

Cette étape d'acclimatation peut comprendre le transfert des plantules dans des godets contenant du terreau, disposés en mini-serre, et exposés à une alternance jour/nuit et une température de l'ordre de 18 à 20°C par exemple.

Une fois que les plantules ont bien repris, elles peuvent être rempotées en pots plus grands avec du terreau de culture et mises en serre.

Une fois les plantes de tournesol fleuries, on veillera à éviter toute pollinisation croisée et on pourra favoriser l'autofécondation, par exemple en disposant des sachets sur les capitules.

Une telle étape d'autofécondation peut être comprise dans la méthode selon l'invention et intervenir après l'obtention des plantes ou plantules haploïdes doublées, et ce avec ou sans étape d'acclimatation.

La présente description divulgue également une méthode telle que décrite ci-avant, comprenant une étape supplémentaire, après l'étape e), d'autofécondation des plantes de tournesol haploïdes doublées obtenues afin d'obtenir des graines homozygotes.

Alternativement, une étape de fécondation, en particulier dirigée, par du pollen d'une autre plante de tournesol peut aussi être comprise dans la méthode divulguée ci-dessus et intervenir après l'obtention des plantes ou plantules haploïdes doublées, et ce avec ou sans étape d'acclimatation.

La présente description divulgue donc également une méthode selon l'un des modes réalisation décrits plus avant, comprenant une étape supplémentaire, après l'étape e), de fécondation par du pollen d'une autre plante de tournesol, des plantes ou plantules haploïdes doublées, afin d'obtenir des graines hétérozygotes.

Enfin, la présente description vise encore l'utilisation des graines de tournesol homozygotes ou des graines de tournesol hétérozygotes obtenues par une méthode selon la présente invention, dans un programme de création et de sélection de nouvelles plantes de tournesol. Un tel programme de création et de sélection peut comprendre des croisements des plantes homozygotes ou hétérozygotes issues de ces graines de tournesol homozygotes ou de ces graines de tournesol hétérozygotes avec d'autres plantes de tournesol homozygotes ou hétérozygotes.

Comme indiqué, une méthode pourra comprendre l'étape supplémentaire d'autofécondation des plantes de tournesol haploïdes doublées afin d'obtenir des graines homozygotes.

Après floraison, on compte en général 4 à 6 semaines pour la récolte de graines qui seront donc homozygotes et qui permettront de reproduire et de fixer ce génotype homozygote, et qui pourront être utilisées en sélection variétale.

### Modes de réalisation de l'invention

Selon un premier mode de réalisation, l'invention vise une méthode de production de plante ou plantule de tournesol (*Helianthus annuus*) haploïde ou haploïde doublée comprenant les étapes suivantes :
- a) Fourniture d'une plantule de tournesol diploïde, au stade de floraison du capitule,
- b) Castration avant émission du pollen, en particulier par ablation des anthères des fleurs tubulées,
- c) prélèvement d'un embryon haploïde immature formé dans l'ovaire non fécondé des fleurs tubulées, sur la plante,
- d) Sauvetage de l'embryon haploïde immature in vitro,
- e) Obtention de plante ou plantule de tournesol haploïde ou haploïde doublée ; la méthode étant caractérisée par l'absence de contact des fleurs tubulées avec du pollen de tournesol (*Helianthus annuus*).

Selon un autre mode de réalisation l'invention vise une méthode selon le premier mode de réalisation, caractérisée en ce que la plantule de l'étape a) est homozygote ou hétérozygote.

Selon un troisième mode de réalisation l'invention vise une méthode selon l'un des modes précédents, caractérisée en ce que l'étape de castration est répétée quotidiennement plusieurs jours, et ce au fur et à mesure du développement des fleurs tubulées,
Selon un quatrième mode de réalisation, l'invention vise une méthode selon le troisième mode de réalisation, caractérisée en ce que la castration est répétée quotidiennement pendant 2 à 7 jours.

Dans un cinquième mode de réalisation, l'invention vise une méthode selon l'un des modes de réalisation précédents, caractérisée en ce que l'embryon haploïde immature est formé dans l'ovaire non fécondé, sur la plante.

Dans un sixième mode de réalisation, l'invention vise une méthode selon l'un des modes de réalisation précédents, caractérisée en ce que les fleurs tubulées non ouvertes après l'étape de castration, sont éliminées avant l'étape de prélèvement de l'embryon immature.

Dans un septième mode de réalisation, l'invention vise une méthode selon l'un des modes de réalisation précédents, caractérisée en ce qu'elle comprend une étape de traitement de doublement chromosomique.

Selon un mode de réalisation de l'invention, le traitement de doublement chromosomique peut comprendre l'application d'un agent de doublement chromosomique au niveau de l'embryon obtenu à l'étape d).

Selon un mode de réalisation de l'invention, le traitement de doublement chromosomique peut comprendre l'application d'un agent de doublement chromosomique au niveau du méristème apical de la plantule obtenue à l'étape e).

Selon un mode de réalisation de l'invention, le traitement de doublement chromosomique peut aussi comprendre l'application d'un agent de doublement chromosomique au niveau des racines de la plantule obtenue à l'étape e).

### EXEMPLES

### Préparation des pieds

Les graines de plantes de tournesol hydrides ou lignées sont semées en godet avec terreau de semis. Les jeunes plants sont rempotés en pots de 7,5 litres avec du terreau.

Les plants sont disposés sous serre selon une photopériode de 16 heures à une température de 18°C environ. Les lampes sont éteintes lorsque les capitules sont bien développés et ouverts, c'est-à-dire que les fleurs ligulées sont apparentes.

### Castration

Le premier jour de castration :
- Retirer manuellement toutes les fleurs ligulées du capitule
- A l'aide d'une pince, retirer tous les fleurons du contour du capitule déjà ouverts. Attention à bien retirer l'ovaire également

Tous les jours de castration :
- Les anthères sorties le matin même sont retirées à l'aide d'une pince. Attention à ne pincer que le bout de l'anthère pour ne pas ôter le pistil ! Plusieurs fleurons peuvent être castrés en même temps.
- Tous les fleurons encore fermés, situés au centre du capitule, sont ôtés (bien retirer l'ovaire).

### Sauvetage embryon

- Récolter les capitules 11 à 15 jours après castration ;
- Egrainer les capitules au laboratoire et placer les ovaires dans des Erlenmeyers différents pour chaque capitule ;
- Désinfecter les ovaires à l'hypochlorite de calcium 5% pendant 15 minutes ;
- Rincer les ovaires 3 fois pendant 5 minutes à l'eau distillée stérile ;
- Ouvrir les ovaires sous la loupe binoculaire et sortir l'embryon s'il est présent ;
- Poser l'embryon sur le milieu solide dont la composition est celle décrite par Todorova et al. 1997.

### Cytométrie

Tester le niveau de ploïdie des plantules prêtes à être acclimatées par cytométrie en flux. Les plantules doivent avoir des racines, une tige et 1 ou 2 étages foliaires environ. Les plantules sont sorties assez tôt d'in vitro pour ne pas qu'elles se nécrosent.

### Doublement Chromosomique

Les plants analysés haploïdes par cytométrie en flux peuvent être traités à la colchicine afin de doubler leur stock chromosomique.

Deux zones du plant peuvent être traitées : les racines ou le méristème apical.

Les racines sont traitées suivant les modalités d'essai entre 2 et 5h à des doses de colchicine comprises entre 300 et 500 mg/L.

Le méristème apical est traité pendant 5h avec une goutte de colchicine à 1500 mg/L.

### Acclimatation

Acclimater les plantules en godets dans du terreau de semis en mini-serres en chambre de culture à 18°C nuit et 20°C jour. A l'apparition des racines sous le godet, les transférer en serre d'abord sous un voile et les rempoter avec du terreau de culture. Au bout de quelques jours, retirer le voile placé sur les plants. Lors de la floraison placer des sachets sur les plants afin d'éviter les contaminations polliniques et favoriser l'autofécondation.

### Récolte des graines

Après la floraison, compter 5 semaines avant la récolte des graines. Placer les graines dans des petits sachets. Identifier chaque sachet avec le numéro du plant dont proviennent les graines et la date de récolte. Les graines peuvent être stockées puis semées et utilisées dans un programme de croisement et sélection.

La durée entre le semis des plantes et l'obtention de graines homozygotes, en passant par la castration, le sauvetage d'embryon, le doublement chromosomique, est de l'ordre de 5 mois. Cela est à comparer avec une série d'au moins 6 à 8 autofécondations nécessaires pour obtenir un niveau d'homozygotie similaire ce qui peut prendre au moins 2 ans.

Le Tableau 1 ci-après résume les résultats obtenus avec des plantes de tournesol diploïdes de départ, en ce qui concerne l'obtention de plantes haploïdes doublées via la méthode décrite ici.

**[Table 1]**

| Plante | NB embryons / capitule | % graines avec embryon | % donnant une plantule | % survie en serre | % 2n spontanés | % plantes fertiles (haploïde s doublées) |
|---|---|---|---|---|---|---|
| Hyb A | 30 | 5 % | 14 % | 18 % | 6 % | 5 % |
| Hyb B | 20 | 3 % | 19 % | 39 % | 19 % | 37 % |
| Hyb C | 6 | 2 % | 29 % | 25 % | 8 % | 25 % |
| Hyb D | 5 | 1 % | 52 % | 45 % | 20 % | 40 % |
| Hyb E | 4 | 1 % | 12 % | 25 % | 0 % | 25 % |
| Hyb F | 18 | 2 % | 14 % | 18 % | 13 % | 15 % |

On constate qu'il est possible d'obtenir entre 5 et 40 % de plantes haploïdes doublées avec des taux de doublement spontané de 6 à 20%.

Ces résultats démontrent que la méthode selon l'invention permet d'obtenir simplement et rapidement des plantes de tournesol haploïdes doublées de manière simple et en s'affranchissant d'une induction avec du pollen.

### Essais avec pollen irradié

Une expérience utilisant du pollen irradié a été menée afin de comparer l'efficacité de la présente invention et de valider le concept de celle-ci.

Le tableau 2 ci-dessous montre les résultats en % de graines présentant un embryon haploïde immature dans des un cas sans pollinisation (c'est-à-dire selon l'invention et telle que présenté dans l'exemple ci-avant) et trois cas avec des lots de grains de pollen irradié selon des techniques connues de l'art antérieur (cf Todorova et al, HELIA, 22, Nr 31, 66 49-56, 1999 ; Todorova et al, Euphytica 97: 249-254, 1997).

**[Table 2]**

| Modalité / Pollen ou non pollinisé | Nombre de capitules testés | % de graines contenant un embryon immature |
|---|---|---|
| Non Pollinisé (invention) | 17 | 6,71% |
| Pollen 1 irradié | 8 | 6,80% |
| Pollen 2 irradié | 46 | 3,70% |
| Pollen 3 irradié | 18 | 4,16% |
| **Total général** | **89** | **4,65%** |

Ces résultats démontrent que contrairement aux préjugés de l'art antérieur, l'induction par contact avec du pollen inactif (irradié) n'est pas un prérequis et ne modifie pas l'induction de graines contenant un embryon immature haploïde.

## Revendications

1. Méthode de production de plante ou plantule de tournesol (*Helianthus annuus*) haploïde ou haploïde doublé comprenant les étapes suivantes :
a) Fourniture d'une plantule de tournesol diploïde, au stade de floraison du capitule,
b) Castration avant émission du pollen, en particulier par ablation des anthères des fleurs tubulées,
c) prélèvement d'un embryon haploïde immature formé dans l'ovaire non fécondé des fleurs tubulées, sur la plante,
d) Sauvetage de l'embryon haploïde immature in vitro,
e) Obtention de plante ou plantule de tournesol haploïde ou haploïde doublé,
la méthode étant **caractérisée par** l'absence de contact des fleurs tubulées avec du pollen de tournesol (*Heliantus annuus*).

2. Méthode selon la revendication 1, **caractérisée en ce que** la plantule de l'étape a) est homozygote ou hétérozygote.

3. Méthode selon l'une des revendications précédentes, **caractérisée en ce que** l'étape de castration est répétée quotidiennement plusieurs jours, et ce au fur et à mesure du développement des fleurs tubulées.

4. Méthode selon la revendication 3, **caractérisée en ce que** la castration est répétée quotidiennement pendant 2 à 7 jours.

5. Méthode selon l'une des revendications précédentes, **caractérisée en ce que** les fleurs tubulées non ouvertes après l'étape de castration, sont éliminées avant l'étape de prélèvement de l'embryon immature.

6. Méthode selon l'une des revendications précédentes, **caractérisée en ce qu'**elle comprend une étape de traitement de doublement chromosomique.

7. Méthode selon la revendication 6, **caractérisée en ce que** le traitement de doublement chromosomique comprend l'application d'un agent de doublement chromosomique au niveau de l'embryon obtenu à l'étape d).

8. Méthode selon la revendication 6, **caractérisée en ce que** le traitement de doublement chromosomique comprend l'application d'un agent de doublement chromosomique au niveau du méristème apical de la plantule obtenue à l'étape e).

9. Méthode selon la revendication 6, **caractérisée en ce que** le traitement de doublement chromosomique comprend l'application d'un agent de doublement chromosomique au niveau des racines de la plantule obtenue à l'étape e).

## Patentansprüche

1. Verfahren zur Herstellung einer haploiden oder haplodiploiden Sonnenblumenpflanze oder eines Sonnenblumenkeimlings (*Helianthus annuus*), umfassend die folgenden Schritte:
a) Bereitstellen eines diploiden Sonnenblumenkeimlings im Stadium der Blütenkopfbildung,
b) Kastrieren vor der Pollenabgabe, insbesondere durch Entfernen der Antheren der röhrenförmigen Blüten,
c) Entnehmen eines im unbefruchteten Fruchtknoten der röhrenförmigen Blüten gebildeten unreifen haploiden Embryos aus der Pflanze,
d) Rettung des unreifen haploiden Embryos in vitro,
e) Gewinnung einer haploiden oder haplodiploiden Sonnenblumenpflanze oder eines Sonnenblumenkeimlings,
wobei das Verfahren **dadurch gekennzeichnet ist, dass** die röhrenförmigen Blüten nicht mit Sonnenblumenpollen (*Heliantus annuus*) in Kontakt kommen.

2. Verfahren nach Anspruch 1, **dadurch gekennzeichnet, dass** der Keimling aus Schritt a) homozygot oder heterozygot ist.

3. Verfahren nach einem der vorstehenden Ansprüche, **dadurch gekennzeichnet, dass** der Kastrationsschritt täglich über mehrere Tage hinweg wiederholt wird, und zwar entsprechend der Entwicklung der röhrenförmigen Blüten.

4. Verfahren nach Anspruch 3, **dadurch gekennzeichnet, dass** die Kastration täglich über einen Zeitraum von 2 bis 7 Tagen wiederholt wird.

5. Verfahren nach einem der vorstehenden Ansprüche, **dadurch gekennzeichnet, dass** die nach dem Kastrationsschritt nicht geöffneten röhrenförmigen Blüten vor dem Schritt der Entnahme des unreifen Embryos entfernt werden.

6. Verfahren nach einem der vorstehenden Ansprüche, **dadurch gekennzeichnet, dass** es einen Schritt der Chromosomenverdopplungsbehandlung umfasst.

7. Verfahren nach Anspruch 6, **dadurch gekennzeichnet, dass** die Chromosomenverdopplungsbehandlung die Anwendung eines Chromosomenverdopplungsmittels auf den in Schritt d) erhaltenen Embryo umfasst.

8. Verfahren nach Anspruch 6, **dadurch gekennzeichnet, dass** die Chromosomenverdopplungsbehandlung die Anwendung eines Chromosomenverdopplungsmittels auf das apikale Meristem des in Schritt e) erhaltenen Keimlings umfasst.

9. Verfahren nach Anspruch 6, **dadurch gekennzeichnet, dass** die Chromosomenverdopplungsbehandlung die Anwendung eines Chromosomenverdopplungsmittels auf die Wurzeln des in Schritt e) erhaltenen Keimlings umfasst.

## Claims

1. A method of producing a haploid or doubled haploid sunflower (*Helianthus annuus*) plant or seedling comprising the following steps:
a) Supply of a diploid sunflower seedling at the flowering stage of the flower head,
b) Castration before pollen emission, in particular by removal of the anthers of tubular flowers,
c) removal of an immature haploid embryo formed in the unfertilized ovary of tubular flowers from the plant,
d) Rescue of the immature haploid embryo in vitro,
e) Obtaining a haploid or doubled haploid sunflower plant or seedling,
the method being **characterized by** the absence of contact between the tubular flowers and sunflower (*Heliantus annuus*) pollen.

2. Method according to claim 1, **characterized in that** the seedling of step a) is homozygous or heterozygous.

3. Method according to one of the preceding claims, **characterized in that** the castration step is repeated daily for several days as the tubular flowers develop.

4. Method according to claim 3, **characterized in that** castration is repeated daily for 2 to 7 days.

5. Method according to one of the preceding claims, **characterized in that** the unopened tubular flowers after the castration step are eliminated before the immature embryo removal step.

6. Method according to one of the preceding claims, **characterized in that** it comprises a chromosome doubling treatment step.

7. The method of claim 6, wherein the chromosome doubling treatment comprises applying a chromosome doubling agent to the embryo obtained in step d).

8. Method according to claim 6, **characterized in that** the chromosome doubling treatment comprises the application of a chromosome doubling agent to the apical meristem of the seedling obtained in step e).

9. Method according to claim 6, **characterized in that** the chromosome doubling treatment comprises the application of a chromosome doubling agent to the roots of the seedling obtained in step e).
